# EUROPEAN PATENT APPLICATION

(11) **EP 1 884 258 A1**
(43) Date of publication of application: **06.02.2008**
(21) Application number: 06254061.2
(22) Date of filing: 02.08.2006
(51) Int. Cl.: A61M 25/06, A61M 5/315, A61B 5/00

(54) **Intravenous catheter introducing device with a flashback member**

(71) Applicant: Shue, Ming-Jeng, Hsi Dist, Taichung City (TW); Huang, Deborah, Taichung City (TW); Shue, Phillip, Hsi District, Taichung City (TW)
(72) Inventor: Shue, Ming-Jeng, Hsi Dist, Taichung City (TW); Huang, Deborah, Taichung City (TW); Shue, Phillip, Hsi District, Taichung City (TW)
(74) Representative: Lord, Hilton David

(57) **Abstract**

An intravenous catheter introducing device includes a needle assembly (2) and a flashback member (1) . The flashback member (1) defines a flashback chamber (11) having an accommodation space with a volume that is variable upon application of a manual force to the flashback chamber (11) such that a reduced pressure is created in the accommodation space toplace the flashback chamber (11) in a ready position, where a tip end (212) of the needle assembly (2) can be brought to pierce a patient' s skin for introducing a tubular catheter (23) into the patient's vein, while a biasing force is generated to act on the flashback chamber (11) . A pressure counteracting member (16;14,32;125;654) is disposed to resist the biasing force such that the flashback chamber (11) can be held in the ready position without application of any manual force once the tip end (212) has pierced into the skin in search of the vein, and such that blood flow into the needle assembly (2) is facilitated to enable the user to check the introduction of the catheter (23).

## Description

This invention relates to an intravenous catheter introducing device, more particularly to an intravenous catheter introducing device having a flashback member that can still be held in a ready position, without application of any manual force, once a needle cannula has pierced into a patient' s skin to locate a vein for taking a blood sample.

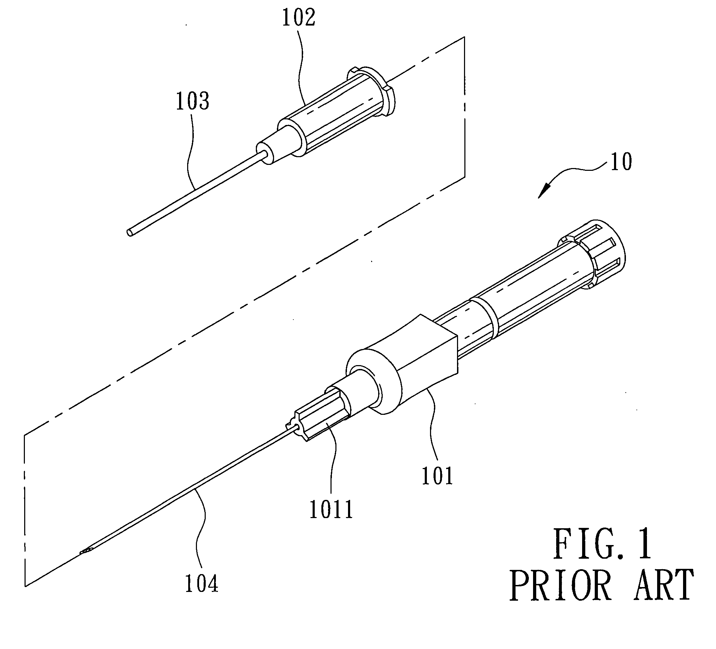

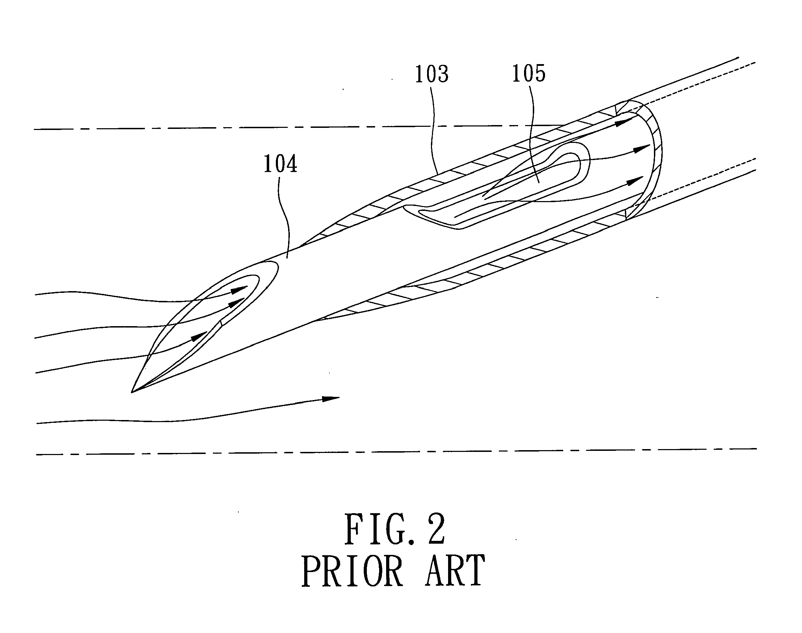

Intravenous catheter inserting devices are generally used to administer medication fluids into or draw blood from a patient's vein. Referring to Figs. 1 and 2, a conventional intravenous catheter inserting device 10 is shown to include a tubular needle seat 101 with a hub end 1011, a needle cannula 104 secured to the hub end 1011, a catheter hub 102 sleeved on the needle seat 1011, and a flexible tubular catheter 103 secured to the catheter hub 102. In use, the catheter 103 and the needle cannula 104 are inserted into the patient' s vein by a health care worker by piercing the patient' s vein with a sharp tip of the needle cannula 104 which projects outwardly of the catheter 103. The health care worker then withdraws the needle cannula 104 from the catheter 103 with one hand and, at the same time, applies pressure to the patient's skin with the other hand, thereby leaving the catheter 103 in the patient's vein. Subsequently, a transfusion member (not shown) with a medication fluid or an empty barrel is connected to the catheter hub 102 for administering the medication fluid into the patient's vein or for drawing blood. At this time, as the health care worker must place the used needle cannula 104 and the needle seat 101 on a tray (not shown) nearby, the exposed sharp tip of the used needle cannula 104 may create a danger of an accidental needle stick. Moreover, blood contamination may occur during connection of the catheter hub 103 to the transfusion member or the empty barrel.

Referring to Fig. 2, during the insertion procedure, the flow of blood into the catheter 103 through a notch 105 in the needle cannula 104 can be observed through the translucent catheter 103. However, due to the presence of the notch 105, the needle cannula 104 tends to be bent during passage of the catheter 103 into the patient's vein. In addition, the blood in the catheter 103 is not visible when the portion of the needle cannula 104 with the notch 105 is inserted into the patient's vein.

Moreover, the conventional intravenous catheter inserting device 10 is specifically not suitable for patients whose blood pressure is not sufficient to permit flow of blood through the notch 105, such as an emergency case, aged people, and pediatrics patients, and patients whose target vein is barely visible due to abundant adipose tissue, such as women and obese patients, since the health care worker will have difficulty determining whether the catheter 103 has been successfully introduced into the target vein, and may need to locate the vein by moving the needle cannula 104 in the skin of the patient, thereby complicating and prolonging the cannulation procedure and causing great discomfort to the patient.

The object of the present invention is to provide an intravenous catheter introducing device having a flashback member that can still be held in a ready position upon insertion of a needle cannula into a patient' s skin for taking a blood sample from a vein so as to facilitate blood flow into the needle cannula to thereby enable the operator to conveniently observe and check the introduction of a catheter.

According to this invention, the intravenous catheter introducing device includes a needle cannula, a flashback member, a pressure counteracting member, a catheter hub, and a tubular catheter. The needle cannula has a front segment terminating at a tip end, and a rear connecting segment opposite to the front segment. The flashback member has front and rear ends opposite to each other in a longitudinal direction, and a surrounding wall which interconnects the front and rear ends. The surrounding wall includes front and rear wall portions proximate to the front and rear ends, respectively. The rear wall portion cooperates with the rear end to define a flashback chamber with an accommodation space confined thereby. The flashback chamber is configured such that the volume of the accommodation space is variable upon application of a manual force to the flashback chamber, and such that, by varying the volume of the accommodation space with the manual force acting on the flashback chamber, a reduced pressure is created in the accommodation space to place the flashback chamber in a ready position, where the tip end is permitted to search for a vein of a patient for taking a blood sample after the tip end has pierced into skin of the patient, while a biasing force acting on the flashback chamber is generated as a result of a pressure difference between the reduced pressure and a pressure of an ambient atmosphere. The front wall portion is associable with the rear connecting segment of the needle cannula such that the flashback chamber is fluidly communicated with the needle cannula. The pressure counteracting member is disposed to resist the biasing force so as to permit the flashback chamber to be held in the ready position without the manual force once the tip end has pierced into the skin in search of the vein. The catheter hub defines therein a duct that permits extension of the front segment of the needle cannula therethrough. The tubular catheter has a proximate segment which is inserted into the duct, and a distal segment which extends from the proximate segment and which surrounds and sheathes the front segment of the needle cannula while permitting the tip end to project forwardly of the distal segment for piercing the skin.

Other features and advantages of the present invention will become apparent in the following detailed description of the preferred embodiments of the invention, with reference to the accompanying drawings, in which:
Fig. 1 is a perspective view of a conventional intravenous catheter inserting device;
Fig. 2 is a partly sectional schematic view illustrating a needle cannula sleeved over by a catheter of the conventional intravenous catheter inserting device;
Fig. 3 is an exploded sectional view of the first preferred embodiment of an intravenous catheter introducing device according to this invention;
Fig. 4 is a sectional view of the first preferred embodiment;
Fig. 5 is a sectional view of the first preferred embodiment when biased;
Fig. 6 is a sectional view of the first preferred embodiment when a catheter is removed;
Fig. 7 is a sectional view of the second preferred embodiment of an intravenous catheter introducing device according to this invention;
Fig. 8 is a sectional view of the third preferred embodiment of an intravenous catheter introducing device according to this invention;
Fig. 9 is a sectional view of the third preferred embodiment when a catheter is removed;
Fig. 10 is an exploded perspective view of the fourth preferred embodiment of an intravenous catheter introducing device according to this invention;
Fig. 11 is a sectional view of the fourth preferred embodiment showing a plunger in a push-out position;
Fig. 12 is a sectional view of the fourth preferred embodiment showing a plunger in a pull-in position;
Fig. 13 is a sectional view of the fourth preferred embodiment when a catheter is removed;
Fig. 14 is an exploded sectional view of the fifth preferred embodiment of an intravenous catheter introducing device according to this invention;
Fig. 15 is a sectional view of the fifth preferred embodiment;
Fig. 16 is a sectional view of the sixth preferred embodiment of an intravenous catheter introducing device according to this invention;
Fig. 17 is a sectional view of the sixth preferred embodiment in a state of use;
Fig. 18 is a sectional view of the sixth preferred embodiment when a catheter is removed;
Fig. 19 is a sectional view of the sixth preferred embodiment having a modified flashback member;
Fig. 20 is a sectional view of the sixth preferred embodiment shown in Fig. 19 and when the flashback member and a catheter are removed;
Fig. 21 is a sectional view of the seventh preferred embodiment of an intravenous catheter introducing device according to this invention;
Fig. 22 is a sectional view of a modified flashback member of the seventh preferred embodiment;
Fig. 23 is a sectional view showing how a reduced pressure in the flashback member shown in Fig. 22 is generated;
Fig. 24 is an exploded perspective view of the eighth preferred embodiment of an intravenous catheter introducing device according to this invention;
Fig. 25 is a sectional view of the eighth preferred embodiment;
Fig. 26 is an exploded perspective view of the ninth preferred embodiment of an intravenous catheter introducing device according to this invention; and
Fig. 27 is a sectional view of the ninth preferred embodiment.

Before the present invention is described in greater detail, it should be noted that same reference numerals have been used to denote like elements throughout the specification.

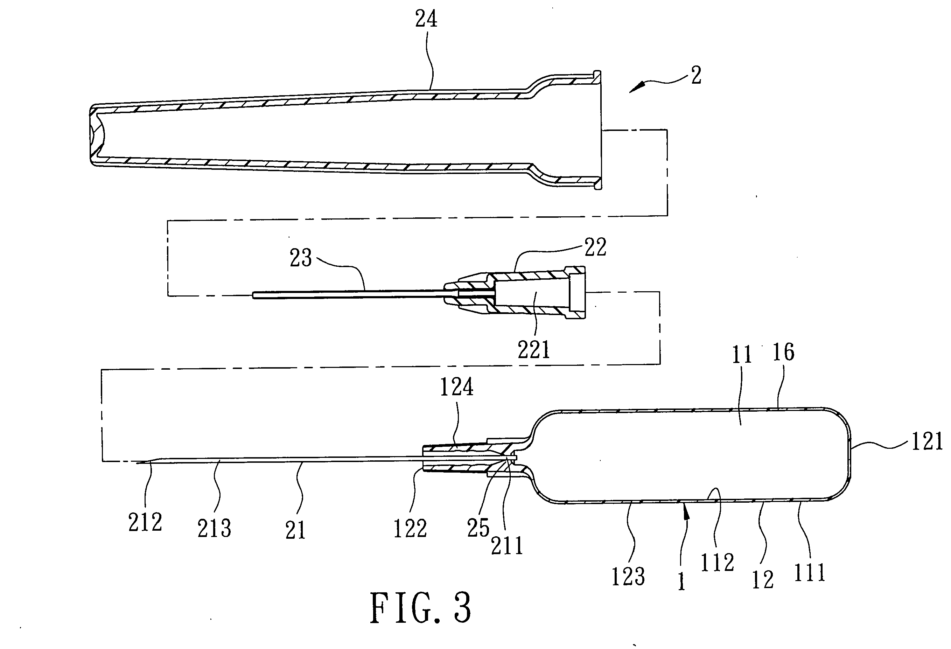

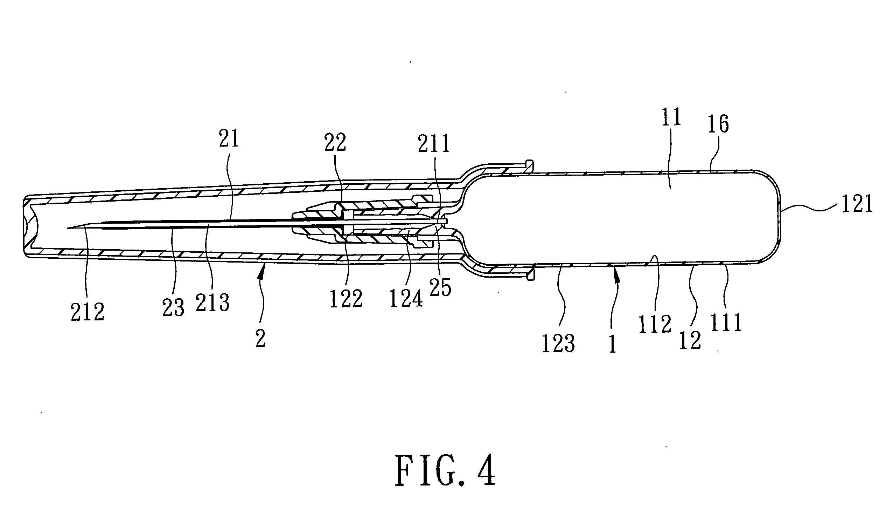

Referring to Figs. 3 to 6, the first preferred embodiment of an intravenous catheter introducing device according to the present invention is shown to comprise a needle assembly 2 and a flashback member 1.

The needle assembly 2 includes a needle cannula 21, a catheter hub 22, a tubular catheter 23, and a tip protector 24. The needle cannula 21 has a front segment 213 terminating at a tip end 212, and a rear connecting segment 211 opposite to the front segment 213. The catheter hub 22 defines a duct 221 therein, which permits extension of the front segment 213 of the needle cannula 21 therethrough. The tubular catheter 23 has a proximate segment which is inserted into the duct 221 of the catheter hub 22, and a distal segment which extends from the proximate segment and which surrounds and sheathes the front segment 213 of the needle cannula 21 while permitting the tip end 212 to project forwardly of the distal segment for piercing the skin of a patient.

The flashback member 1 has front and rear ends 122,121 opposite to each other in a longitudinal direction, and a surrounding wall 12 which interconnects the front and rear ends 122,121. The surrounding wall 12 includes a large-diameter rear wall portion 123 proximate to the rear end 121 and cooperating with the rear end 121 to define a flashback chamber 11 with an accommodation space confined thereby, andasmall-diameterfrontwallportion124 opposite to the rear wall portion 123 in the longitudinal direction and proximate to the front end 122. In this embodiment, a tubular needle seat 25, which is disposed to be surrounded by and which is integrally formed with the front wall portion 124, is configured to secure the rear connecting segment 211 of the needle cannula 21 to the front wall portion 124 so as to permit the needle cannula 21 to be fluidly communicated with the flashback chamber 11.

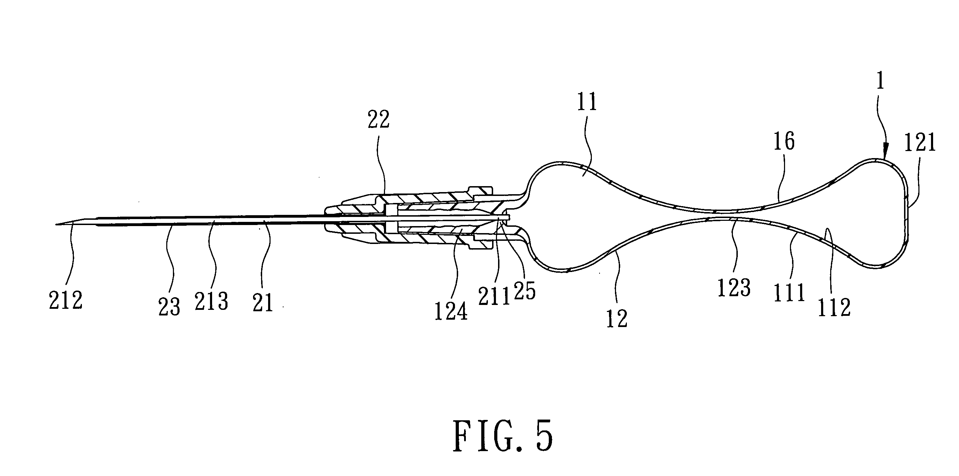

The rear wall portion 123 is integrally formed with the rear end 121. The flashback chamber 21 is made from a translucent deformable material such that the volume of the accommodation space is variable when the flashback chamber 21 is subjected to application of a manual force, and has an outer wall surface 111, and an inner wall surface 112 which is opposite to the outer wall surface 111 and which confronts the accommodation space. A pressure counteracting member 16 is interposed between and is integrally formed with the inner and outer wall surfaces 111,112. Thus, as shown in Fig. 5, by varying the volume of the accommodation space through application of a manual force onto the flashback chamber 11, a reduced pressure is created in the accommodation space to place the flashback chamber 11 in a ready position, where the tip end 212 is permitted to search for a vein of a patient for taking a blood sample after the tip end 212 has pierced into the skin of the patient. To illustrate, when the inner wall surface 112 is moved towards the accommodation space (i.e., the rear wall portion 123 is squeezed and deformed), a first biasing force acting on the flashback chamber 11 is generated as a result of pressure difference between the reduced pressure and a pressure of an ambient atmosphere. The pressure counteracting member 16 acquires an urging force which resists the first biasing force so as to hold the flashback chamber 11 in the ready position without the need for application of a manual force once the tip end 212 has pierced into the skin in search of the vein.

The tip protector 24 is detachably sleeved on the rear wall portion 123 adjacent to the front wall portion 124 for shielding the needle cannula 21, the catheter hub 22 and the tubular catheter 23.

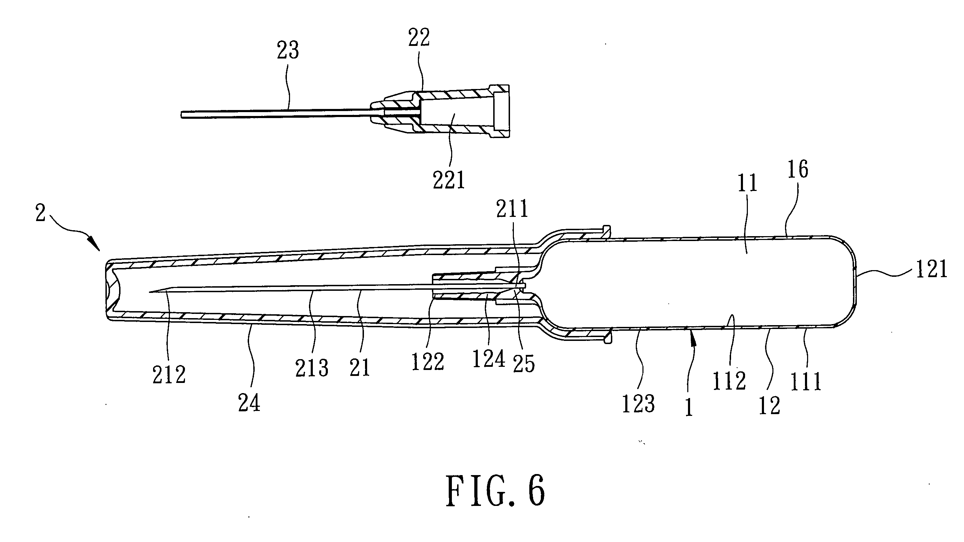

In use, after the tip protector 24 is removed, the user can, with one hand, hold and squeeze the rear wall portion 123, as shown in Fig. 5, and pierce the skin of a patient with the tip end 212. The user can then gradually release the rear wall portion 123 such that the urging force is generated in the counteracting member 16 to facilitate the flow of blood into the flashback chamber 11, thereby permitting the user to check whether the tubular catheter 23 has been successfully introduced into a target vein of the patient. Referring to Fig. 6, the user can separate the catheter hub 22 from the flashback member 1 with the other hand, and sleeve the tip protector 24 on the rear wall portion 123 to shield the used needle cannula 21 for subsequent safe disposal.

It is noted that during operation, once the tip end 212 has pierced into the skin of the patient, the flashback chamber 11 can be held in the ready position as shown in Fig. 5 by the first biasing force without application of any manual force until the blood flows into the flashback chamber 11 through the needle cannula 21 by virtue of the urging force of the pressure counteracting member 16, i.e. , the restoring force of the deformable material of the pressure counteracting member 16. Thus, the catheter introducing operation is convenient to conduct.

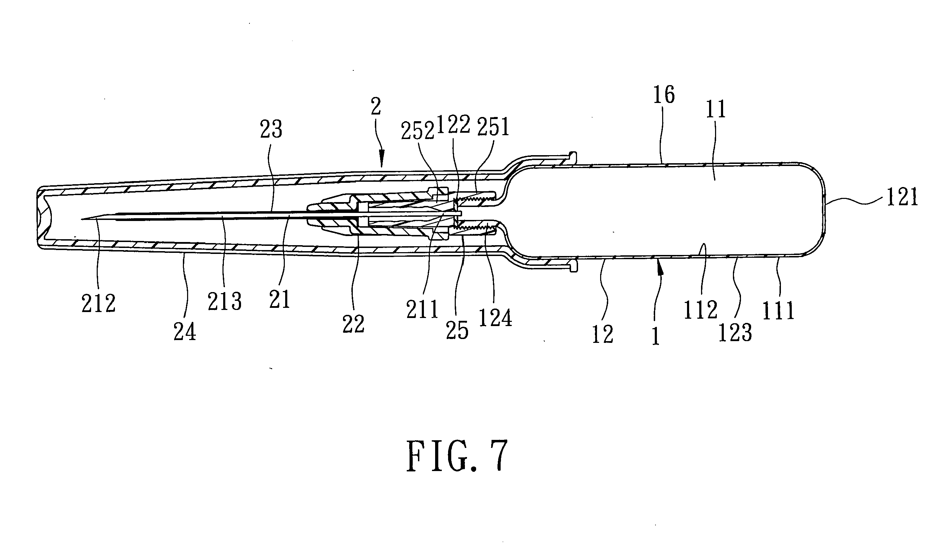

Referring to Fig. 7, the second preferred embodiment of an intravenous catheter introducing device according to this invention is shown to be similar to the first preferred embodiment in construction. The difference resides in that the tubular needle seat 25 has a rear coupling portion 251 which is threadedly engageable with the front wall portion 124 of the flashback member 1 instead of being integrally formed with the front wall portion 124, and a front securing portion 252 which is configured to secure the rear connecting segment 211 of the needle cannula 21 such that the needle cannula 21 is fluidly communicated with the flashback chamber 11. In addition, the catheter hub 22 is detachably sleeved on the front securing portion 252.

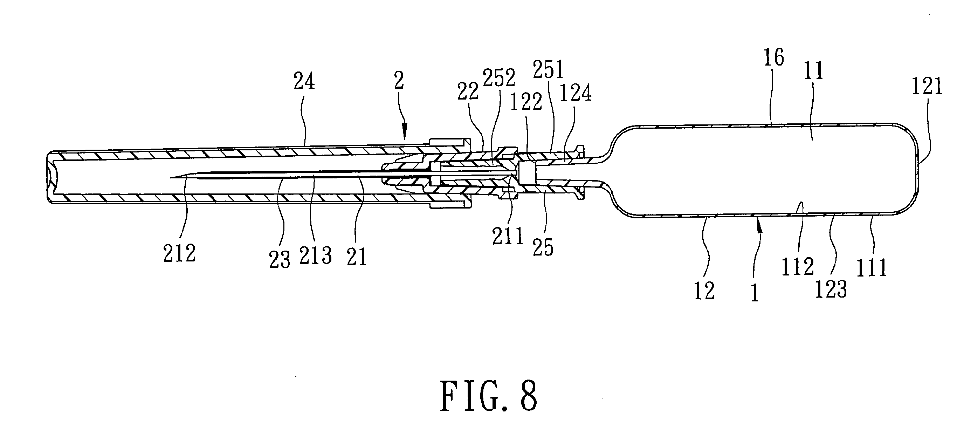

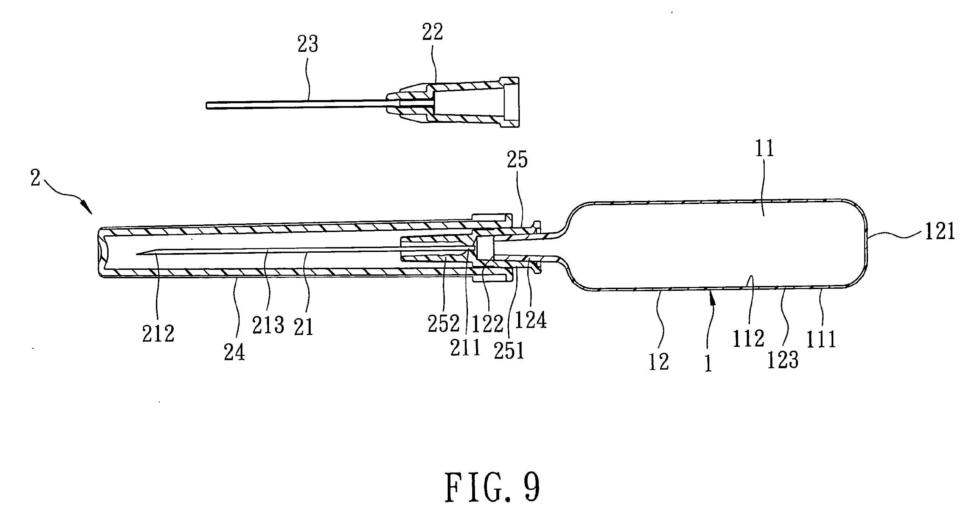

Referring to Figs. 8 and 9, the third preferred embodiment of an intravenous catheter introducing device according to this invention is shown to be similar to the second preferred embodiment in construction. The difference resides in that the rear coupling portion 251 of the needle seat 25 is detachably sleeved on the front wall portion 124 of the flashback member 1. In addition, the tip protector 24 is detachably sleeved on the catheter hub 22 for shielding the needle cannula 21 and the tubular catheter 23 before use. As shown in Fig. 9, the tip protector 24 is configured to be detachably sleeved on the rear coupling portion 251 of the needle seat 25 after the catheter hub 22 is detached from the front securing portion 252, for shielding the used needle cannula 21.

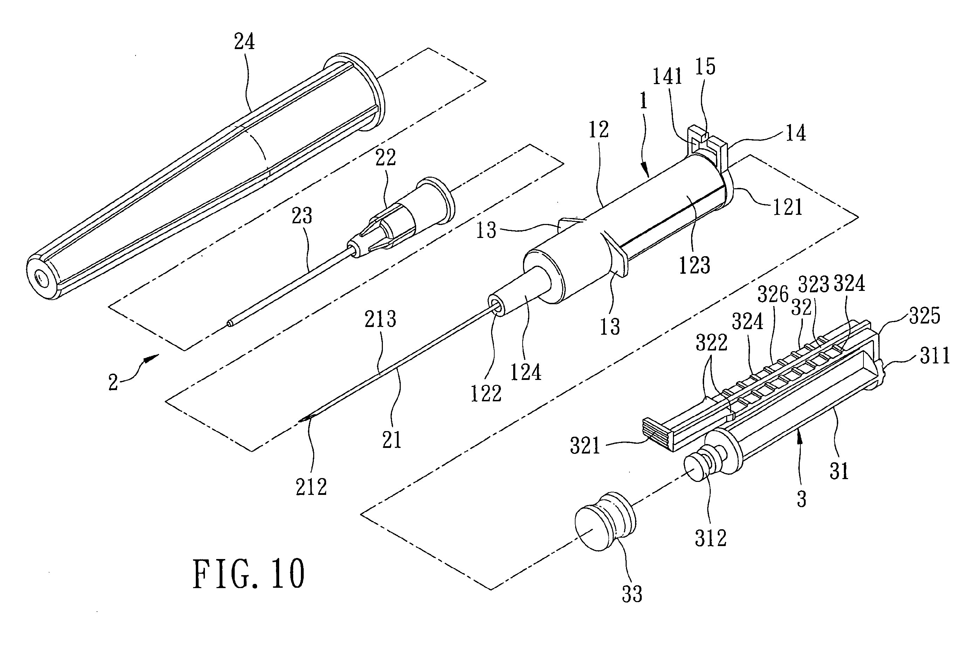

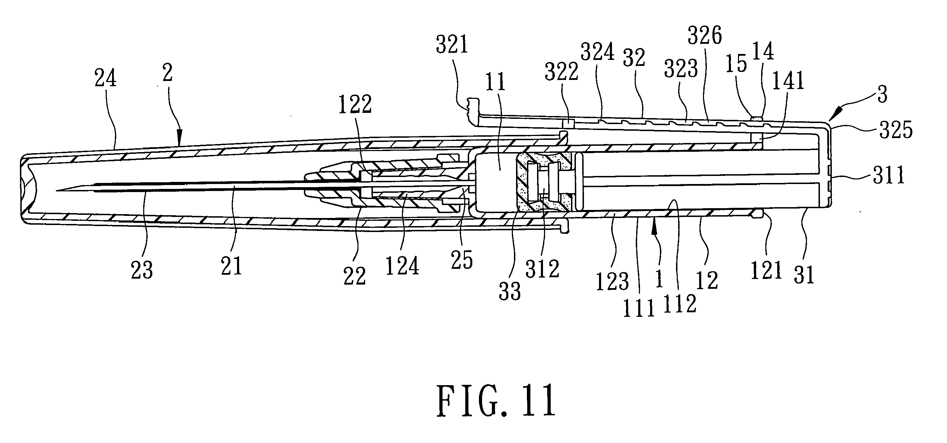

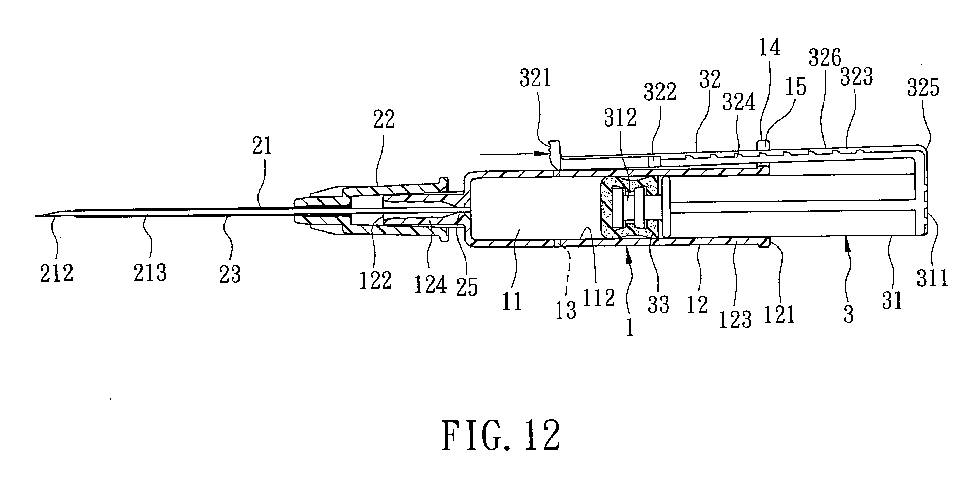

Referring to Figs. 10 to 12, the fourth preferred embodiment of an intravenous catheter introducing device according to this invention is shown to be similar to the first preferred embodiment in construction. The differences reside in that the rear end 121 defines an end opening communicated with the accommodation space. A plunger 3 includes a plunger head 33 and a plunger stem 31. The plunger head 33 is configured to be in fluidly tight contact with, and to be slidable relative to, the inner wall surface 112 of the flashback member 1 in the longitudinal direction. The plunger stem 31 has a securing end 312 fixed to the plunger head 33, and an actuated end 311 which is opposite to the securing end 312 in the longitudinal direction, and which extends outwardly of the end opening of the rear end 121 to be actuated so as to move the plunger head 33 to slide along the inner wall surface 112 from a push-out position, as shown in Fig. 11, where the plunger head 33 is closer to the front wall portion 124, to a pull-in position, as shown in Fig. 12, where the plunger head 33 is remote from the front wall portion 124, thereby generating the first biasing force that biases the plunger head 33 towards the front wall portion 124 once the tip end 212 has pierced into the skin of a patient in search of a vein.

In this embodiment, the pressure counteracting member includes a retaining portion 14 and an actuator 32. The retaining portion 14 is fixed to the outer wall surface 111 adjacent to the rear end 121 of the flashback member 1, and has a confining hole 141 extending therethrough in the longitudinal direction, and an engaging slot 15 which is communicated with the confining hole 141 in a transverse direction relative to the longitudinal direction. The actuator 32 has a rear fixed end 325 which is integrally formed with the actuated end 311, a tongue portion 326 which extends forwardly from the rear fixed end 325 to extend through the confining hole 141 and which terminates at a front pushed end 321, a plurality of retained regions 324 which are in the form of protrusions disposed on the tongue portion 326 and which are spaced apart from one another in the longitudinal direction, and a longitudinally extending reinforcing ridge 323 which is disposed on the tongue portion 326 to increase the stiffness of the tongue portion 326. The tongue portion 326 is configured to acquire a second biasing force that biases the tongue portion 326 to a retained position, as shown in Fig. 11, where a selected one of the retained regions 324 is engaged with the retaining portion 14 to resist the first biasing force so as to prevent the plunger head 33 from being shifted relative to the inner wall surface 112 while the reinforcing ridge 323 engages the engaging slot 15. The tongue portion 326 is displaceable relative to the plunger stem 31 by a manual force to a released position, as shown in Fig. 12, where the tongue portion 326 is released from the retaining portion 14 so as to permit the sliding movement of the plunger head 33 along the inner wall surface 112.

The actuator 32 further has a pair of limiting barriers 322 which are disposed between the retained regions 324 and the front pushed end 321, and which are configured to extend transversely to be blocked by the retaining portion 14 when the tongue portion 326 is in the released position so as to prevent excess rearward movement of the plunger head 33 relative to the inner wall surface 112.

The flashback member 1 has a pair of lugs 13 which are disposed on and which extend from the outer wall surface 111 for gripping by a user's fingers so as to facilitate pulling of the plunger head 33 to the pull-in position.

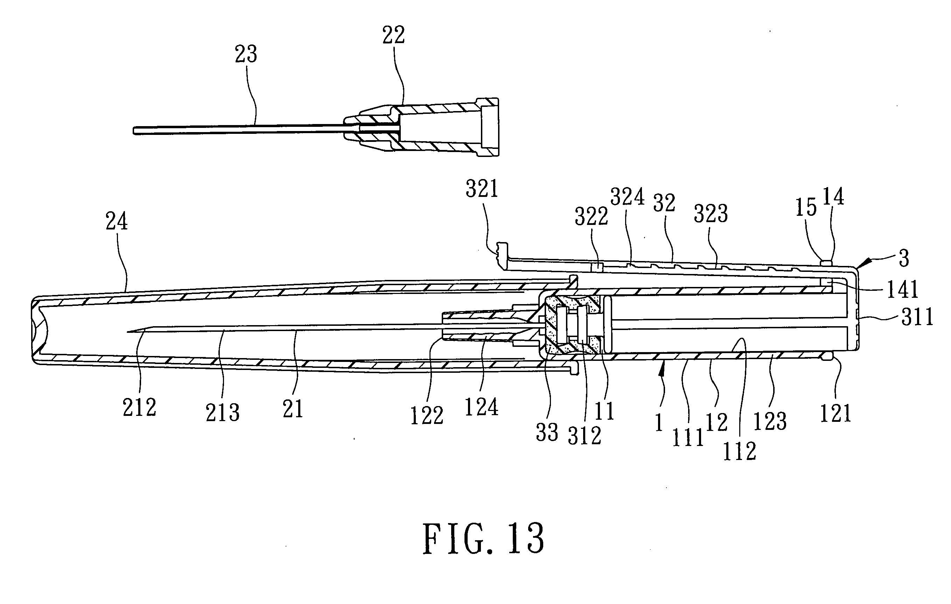

In use, after the tip protector 24 is removed, the user can hold the rear wall portion 123 with one hand, as shown in Fig. 12, and pierce a target vein of a patient with the tip end 212 so that blood flows into the flashback chamber 11 to indicate a successful introduction of the tubular catheter 23. If blood is not observed in the flashback chamber 11, the user can press the lugs 13 with his/her thumb and middle finger, push the front pushed end 321 to displace the tongue portion 326 to the released position, and move the plunger head 33 rearwardly to the pull-in position so as to generate a reduced pressure in the flashback chamber 11 to thereby facilitate flow of blood into the flashback chamber 11. Referring to Fig. 13, the user can then separate the catheter hub 22 from the flashback member 1 with the other hand, and sleeve the tip protector 24 on the rear wall portion 123 to shield the used needle cannula 21 for subsequent safe disposal.

It is noted that during operation, by means of the engagement of one of the retained regions 324 with the retaining portion 14 and by means of the second biasing force of the tongue portion 326, the plunger head 33 can be retained relative to the inner wall surface 112 against the first biasing force without application of any manual force, thereby facilitating the catheter introducing operation.

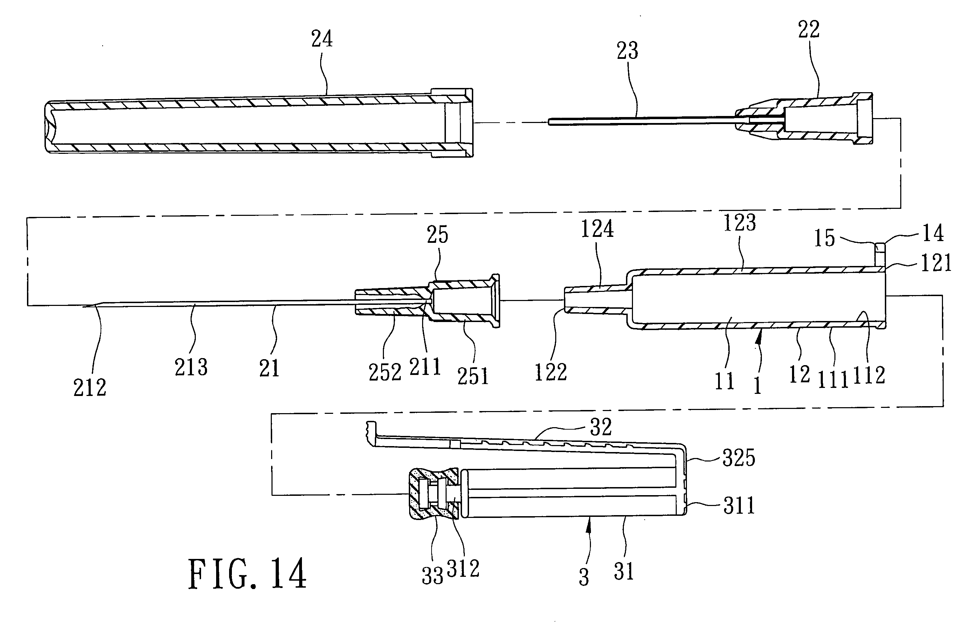

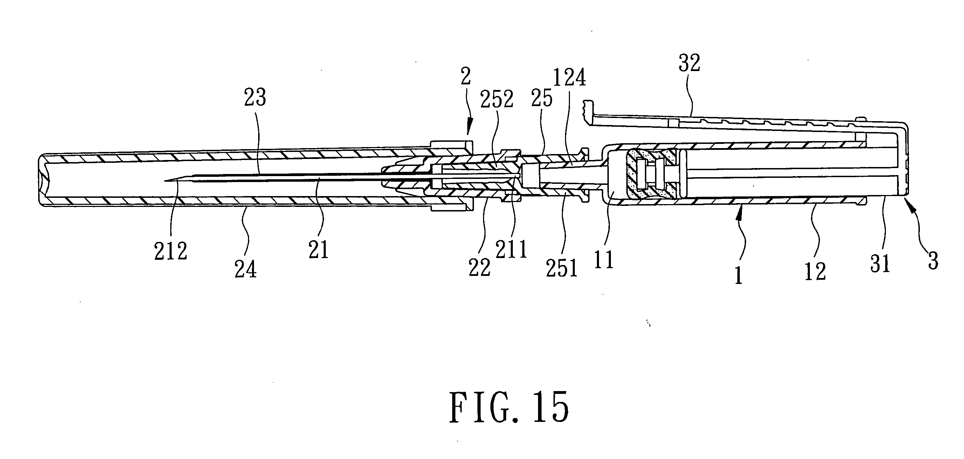

Referring to Figs. 14 and 15, the fifth preferred embodiment of an intravenous catheter introducing device according to this invention is shown to be similar to the fourth preferred embodiment in construction. The difference resides in that the tubular needle seat 25 has a rear coupling portion 251 which is detachably sleeved on the front wall portion 124 instead of being integrally formed with the front wall portion 124, and a front securing portion 252 which is configured to secure the rear connecting segment 211 of the needle cannula 21 such that the needle cannula 21 is fluidly communicated with the flashback chamber 11. In addition, the catheter hub 22 is detachably sleeved on the front securing portion 252. The tip protector 24 is detachably sleeved on the catheter hub 22 for shielding the needle cannula 21 and the tubular catheter 23 before use. As shown in Fig. 15, the tip protector 24 is configured to be detachably sleeved on the rear coupling portion 251 of the tubular needle seat 25 after the catheter hub 22 is detached from the front securing portion 252, for shielding the used needle cannula 21.

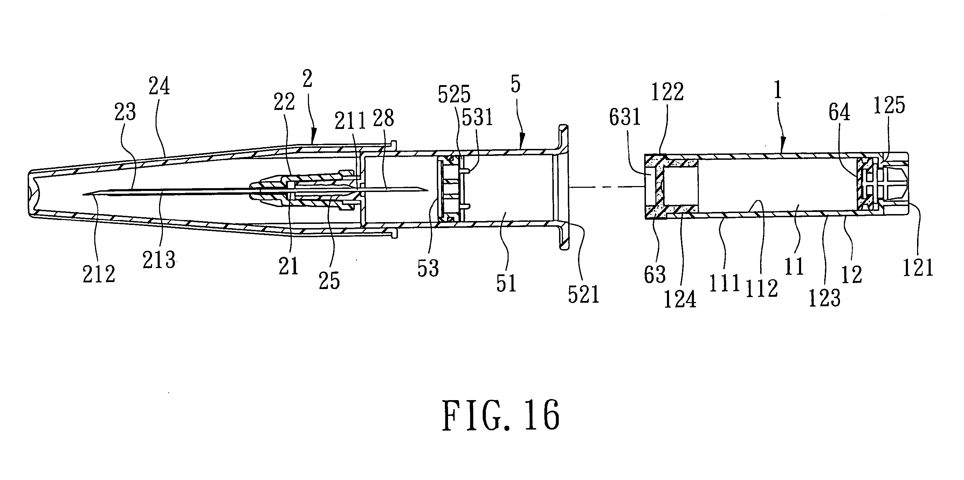

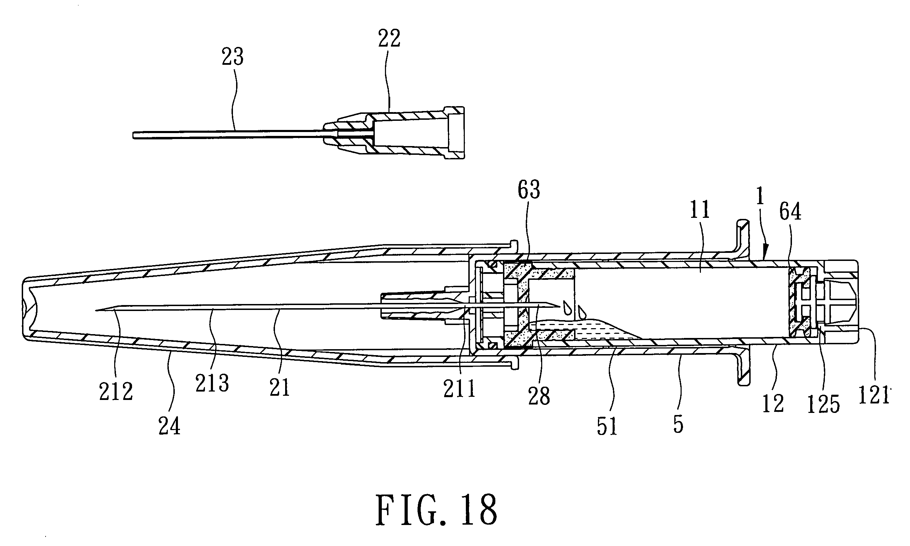

Referring to Figs. 16 to 18, the sixth preferred embodiment of an intravenous catheter introducing device according to this invention is shown to be similar to the first preferred embodiment in construction. In this embodiment, the intravenous catheter introducing device comprises a needle assembly 2, a shell member 5, and a flashback member 1. The needle assembly 2 includes a needle cannula 21, a needle seat 25, a catheter hub 22, a tubular catheter 23, a tip protector 24, and an internal needle 28.

The internal needle 28 is integrally formed with and extends rearwardly from a rear connecting segment 211 of the needle cannula 21 in the longitudinal direction, and terminates at a tip end. The needle seat 25 is disposed to secure the rear connecting segment 211 of the needle cannula 21. The shell member 5 is integrally formed with and extends rearwardly from the needle seat 25 to surround the internal needle 28, and terminates at a rear access end 521, thereby defining a receiving chamber 51 for detachably receiving the flashback member 1 through the rear access end 521.

A partition member 53, which is made from an air-permeable and liquid-impermeable material, is disposed in the receiving chamber 51, and is configured to air-tightly engage and to be movable relative to, the shell member 5. The partition member 53 has an engaging portion 531 formed on a side thereof. An annular barrier 525 extends inwardly and radially from the shell member 5.

A front sealing member 63 is disposed to be in air-tight engagement with the front wall portion 124 of the flashback member 1 so as to seal the front end 122. A rear sealing member 64 is disposed to be in air-tight engagement with the rear wall portion 123 so as to seal the rear end 121, thereby confining the flashback chamber 11. The front sealing member 63 has an engaging recess 631 facing forwardly. Fluid with a reduced pressure is disposed in the flashback chamber 11. An engaging portion 125 is disposed on and extends from the inner wall surface 112 adjacent to the rear end 121 to serve as the pressure counteracting member such that the rear sealing member 64 is blocked by the engaging portion 125 so as tomaintain the flashback chamber 11 with the reduced pressure.

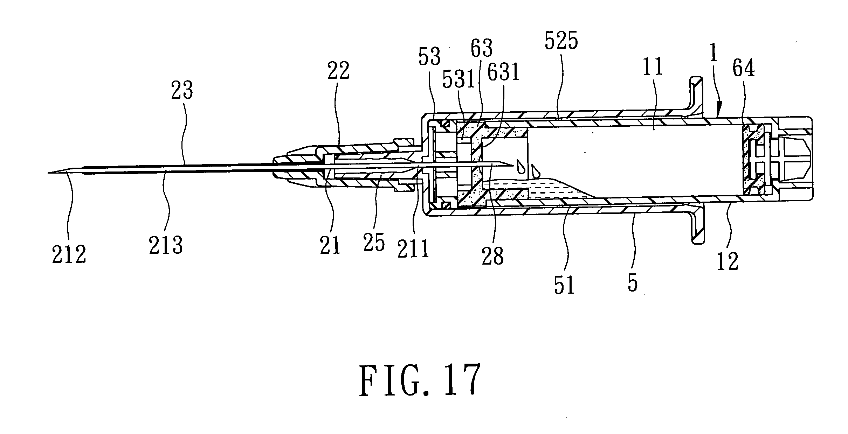

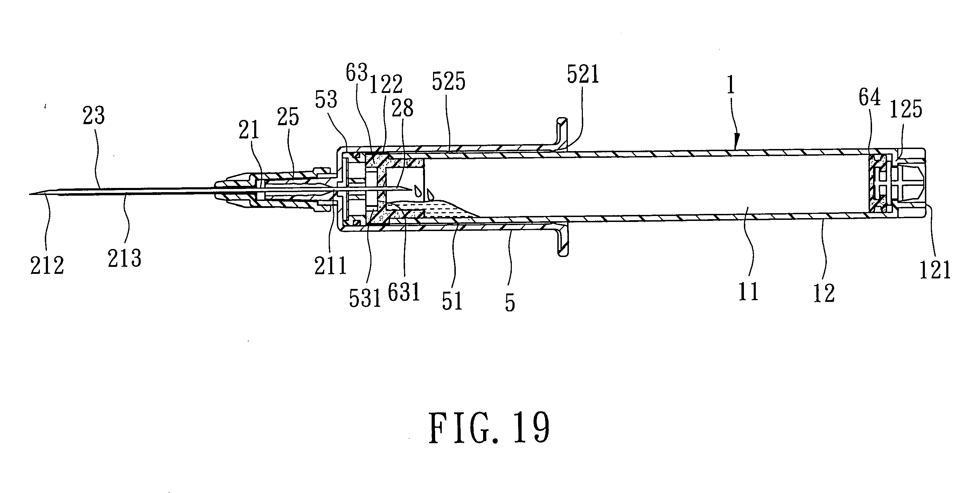

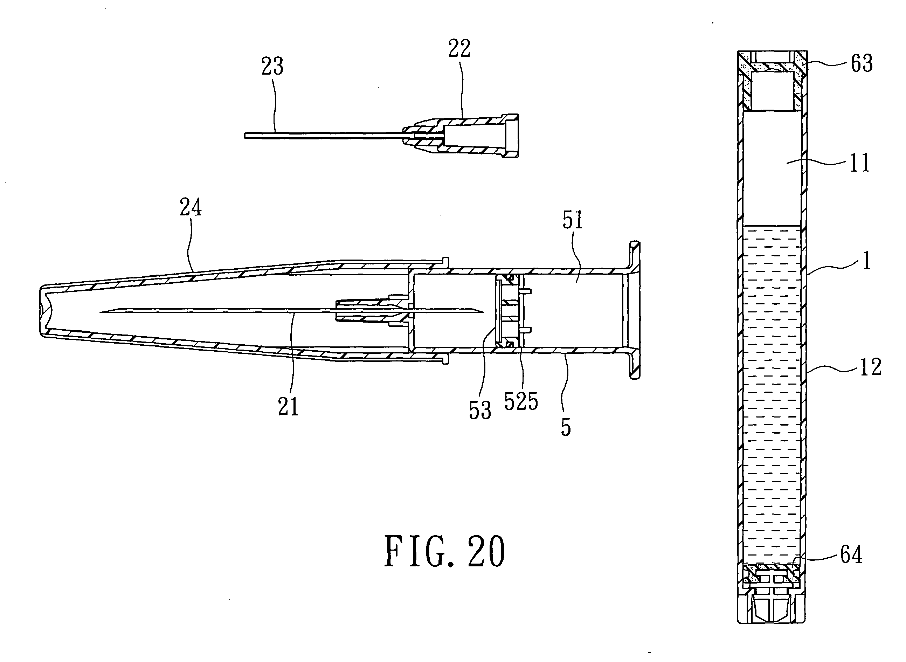

In use, after the tip protector 24 is removed, the user can hold the shell member 5 with one hand, as shown in Fig. 17, and pierce a target vein of a patient with the tip end 212 so that blood flows into the receiving chamber 51 to indicate a successful introduction of the tubular catheter 23. If blood is not observed in the receiving chamber 51, the user can insert the flashback member 1 into the receiving chamber 51 such that the engaging portion 531 engages the engaging recess 631. Subsequently, the partition member 53 is pushed by the flashback member 1 to permit the internal needle 28 to pierce through the partition member 53. A subsequent pushing action of the flashback member 1 results inpassingof the internal needle 28 through the front sealing member 63 so as to fluidly communicate the flashback chamber 11 with the needle cannula 21, thereby facilitating the flow of blood into the flashback chamber 11 by means of the reduced pressure. Moreover, the blood can be collected into the flashback chamber 11 for taking a blood sample. As shown in Figs. 19 and 20, another flashback member 1, which has a longer flashback chamber 11, is used for collecting a larger amount of blood sample as required. After the blood sample is collected, the flashback member 1 can be pulled rearwardly to be removed from the shell member 5. The partition member 53 is prevented from being removed from the shell member 5 through the rear access end 521 by virtue of the annular barrier 525. The flashback member 1, in which the blood sample is collected, can be transferred for the subsequent medical tests. Thus, the risk of pollution and infection and the amount of medical waste can be relatively reduced.

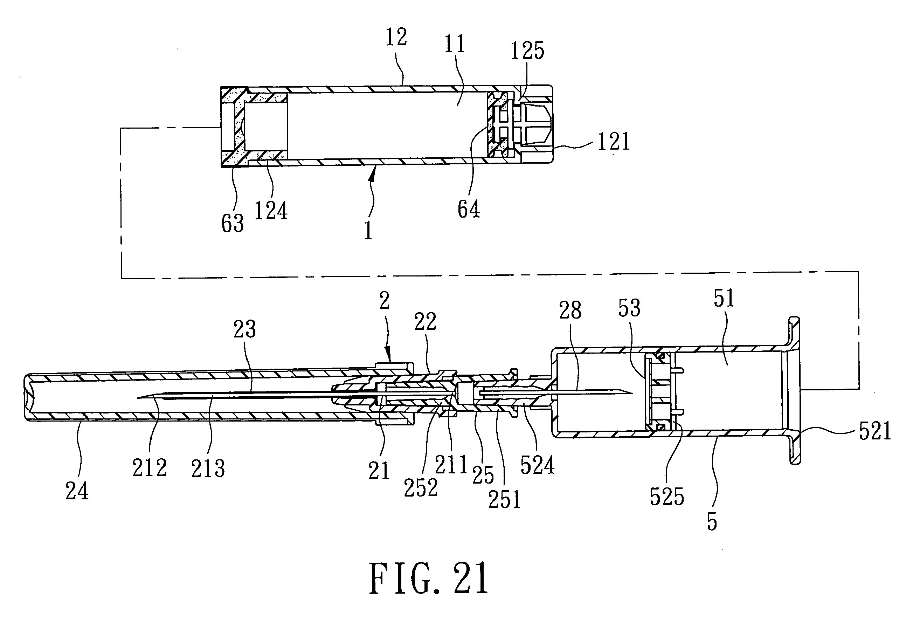

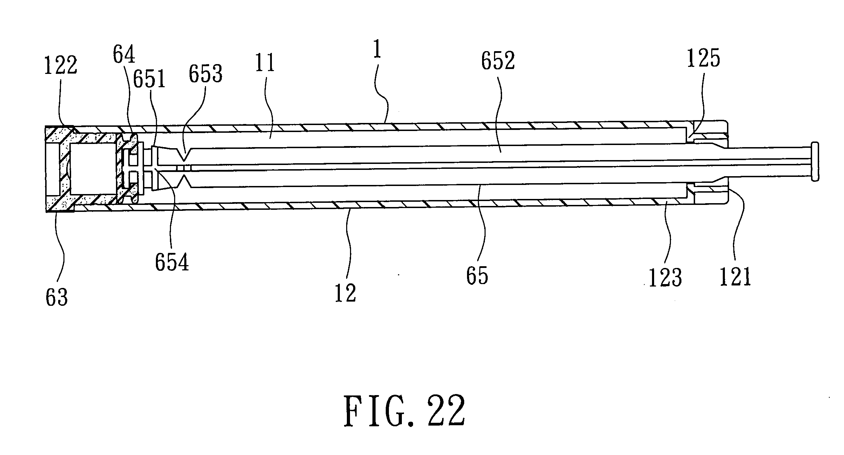

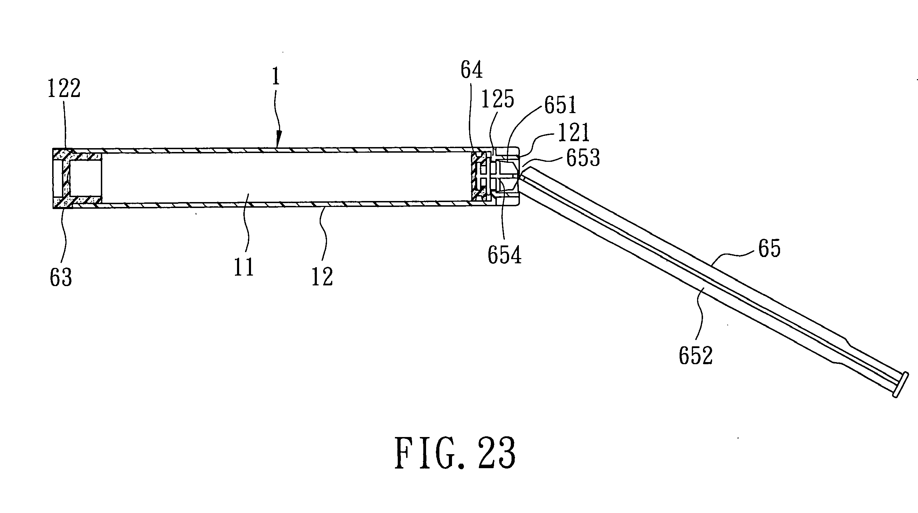

Referring to Figs. 21 to 23, the seventh preferred embodiment of an intravenous catheter introducing device according to this invention is shown to be similar to the sixth preferred embodiment in construction. Instead of being integrally formed with the needle cannula 21, the internal needle 28 of the seventh embodiment is separately formed, and has a first segment which extends into the receiving chamber 51 of the shell member 5 to be surrounded by the shell member 5, and a second segment which is opposite to the first segment and which is surrounded and secured by a needle hub member 524 that extends forwardly from the shell member 5. The needle seat 25 has a front securing portion 252 which is configured to secure the rear connecting segment 211 of the needle cannula 21, and a rear coupling portion 251 which is detachably sleeved on the needle hub member 524 such that the needle cannula 21 is fluidly communicated with the internal needle 28. The catheter hub 22 is detachably sleeved on the front securing portion 252.

Moreover, in order to generate a reduced pressure in the flashback chamber 11, the rear sealing member 64 is disposed to be movable relative to the surrounding wall 12 in the longitudinal direction. A plunger 65 has a head portion 651 which is connected to the rear sealing member 64, and a shank portion 652 which extends rearwardly from the head portion 651 and outwardly of the rear end 121 such that the plunger 65 is pulled rearwardly to move the rear sealing member 64 towards the rear end 121 so as to generate the reduced pressure in the flashback chamber 11. In addition, the shank portion 652 has a weakening region 653 which is formed proximate to the head portion 651 for facilitating breaking of the shank portion 652 after the rearwardly pulling operation of the plunger 65.

In this embodiment, the pressure counteracting member includes first and second engaging portions 125,654 which are disposed on the rear wall portion 123 of the flashback member 1 adjacent to the rear end 121 and the head portion 651, respectively, and which are configured such that a forced movement of the second engaging portion 654 over the first engaging portion 125 by virtue of rearwardly pulling the head portion 651 results in a press-fit engagement between the first and second engaging portions 125,654, thereby preventing a forward movement of the rear sealing member 64 and thereby holding the flashback chamber 11 in the ready position.

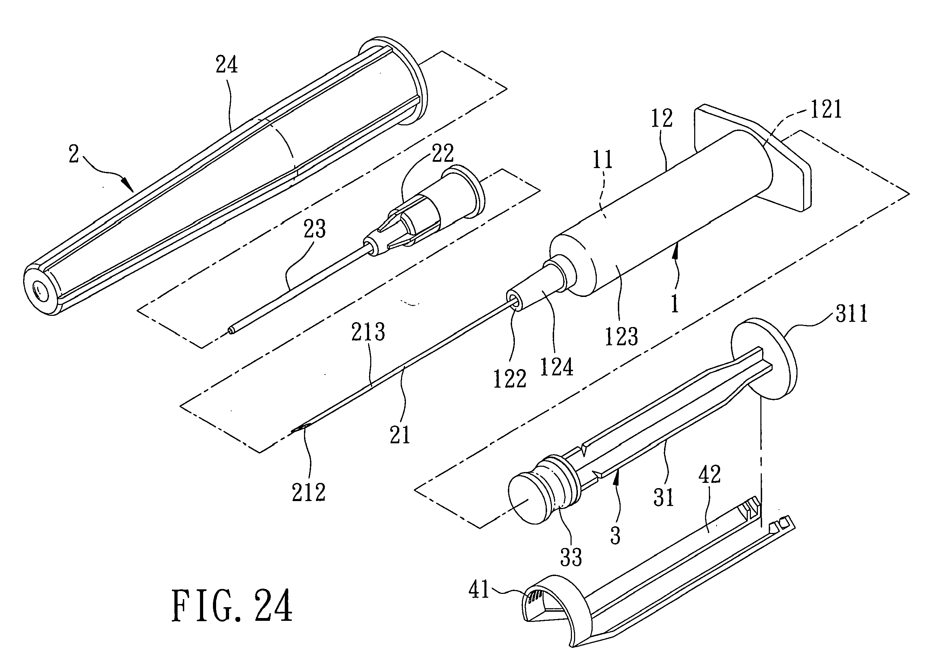

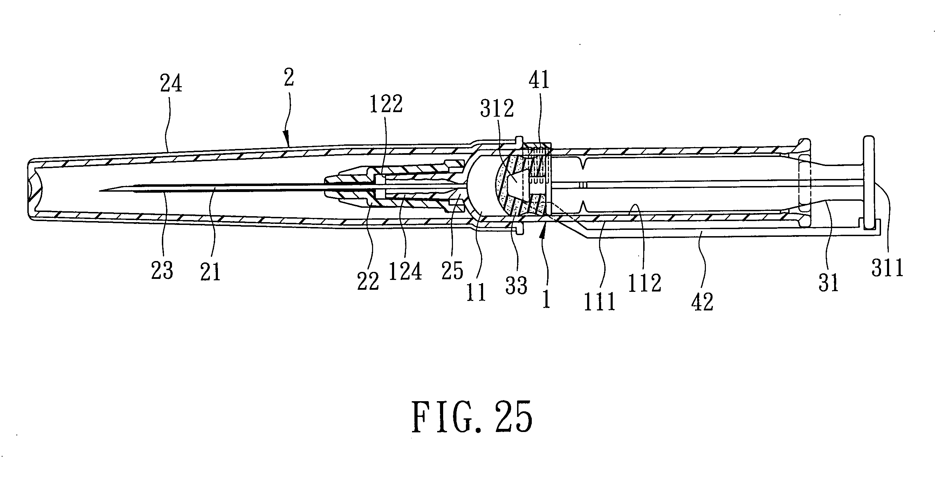

Referring to Figs. 24 and 25, the eighth preferred embodiment of an intravenous catheter introducing device according to this invention is shown to be similar to the fourth preferred embodiment in construction. In this embodiment, the pressure counteracting member includes a traction ring 41 and a connecting bar 42. The traction ring 41 is sleeved on and is in frictional engagement with the outer wall surface 111 of the flashback chamber 11 with a frictional force. The connecting bar 42 is disposed to interconnect the traction ring 41 and the actuated end 311 of the plunger stem 31 so as to permit the traction ring 41 to be moved with the plunger head 3 3 . Thus, once the actuated end 311 is relieved of a manual force immediately after the plunger head 33 is pulled by the manual force against the frictional force to move from the push-out position to the pull-in position to create the reduced pressure, the frictional force can hold the traction ring 41 onto the outer wall surface 111 against the pressure difference, thereby holding the plunger head 33 at the pull-in position.

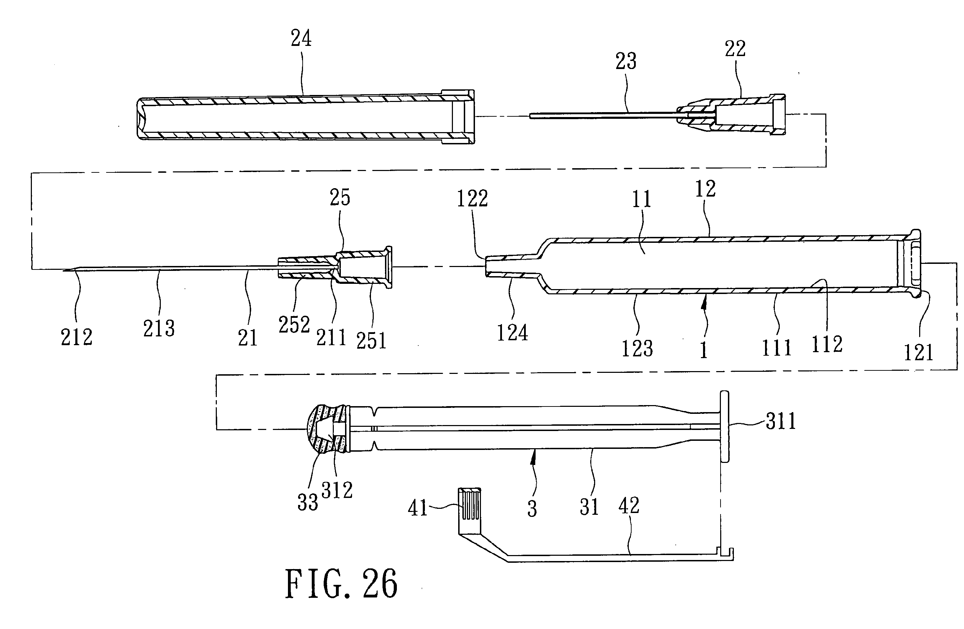

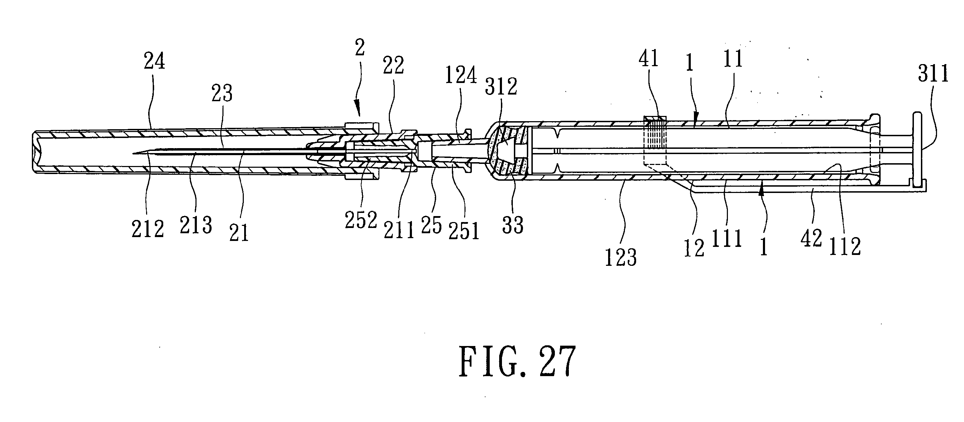

Referring to Figs. 26 and 27, the ninth preferred embodiment of an intravenous catheter introducing device according to this invention is shown to be similar to the eighth preferred embodiment in construction. The difference resides in that the tubular needle seat 25 has a rear coupling portion 251 which is detachably sleeved on the front wall portion 124 instead of being integrally formed with the front wall portion 124, and a front securing portion 252 which is configured to secure the rear connecting segment 211 of the needle cannula 21 such that the needle cannula 21 is fluidly communicated with the flashback chamber 11. In addition, the catheter hub 22 is detachably sleeved on the front securing portion 252. The tip protector 24 is detachably sleeved on the catheter hub 22 for shielding the needle cannula 21 and the tubular catheter 23 before use. The tip protector 24 is configured to be detachably sleeved on the rear coupling portion 251 of the tubular needle seat 25 after the catheter hub 22 is detached from the front securing portion 252, for shielding the used needle cannula 21.

## Claims

1. An intravenous catheter introducing device comprising:
a needle cannula (21) having a front segment (213) terminating at a tip end (212), and a rear connecting segment (211) opposite to said front segment (213);
a catheter hub (22) which defines therein a duct (221) that permits extension of said front segment (213) of said needle cannula (21) therethrough; and
a tubular catheter (23) having a proximate segment which is inserted into said duct (221), and a distal segment which extends from said proximate segment and which surrounds and sheathes said front segment (213) of said needle cannula (21) while permitting said tip end (212) to project forwardly of said distal segment for piercing the skin, **characterized by**:
a flashback member (1) having front and rear ends (122,121) opposite to each other in a longitudinal direction, and a surrounding wall (12) which interconnects said front and rear ends (122, 121), said surrounding wall (12) including
a rear wall portion (123) which is proximate to said rear end (121), and which cooperates with said rear end (121) to define a flashback chamber (11) with an accommodation space confined thereby, said flashback chamber (11) being configured such that volume of said accommodation space is variable subject to application of a manual force to said flashback chamber (11), and such that, by virtue of varying the volume of said accommodation space with the manual force acting on said flashback chamber (11), a reduced pressure is created in said accommodation space to place said flashback chamber (11) in a ready position, where said tip end (212) is permitted to search for a vein of a patient for taking a blood sample after said tip end (212) has pierced into skinof the patient, while a first biasing force acting on said flashback chamber (11) is generated as a result of pressure difference between the reduced pressure and pressure of an ambient atmosphere; and
a front wall portion (124) which is opposite to said rear wall portion (123) in the longitudinal direction, and which is proximate to said front end (122), said front wall portion (124) being associable with said rear connecting segment (211) such that said flashback chamber (11) is fluidly communicated with said needle cannula (21); and
a pressure counteracting member (16;14,32;125;654) disposed to resist said first biasing force so as to permit said flashback chamber (11) to be held in the ready position without the manual force once said tip end (212) has pierced into the skin in search of the vein.

2. The intravenous catheter introducing device of Claim 1, **characterized in that** said rear wall portion (123) is integrally formed with said rear end (121), said flashback chamber (11) havinganouterwall surface (111) and an inner wall surface (112) that is opposite to said outer wall surface (111), and that confronts said accommodation space, said pressure counteracting member (16) being interposed between, and being integrally formed with, said inner and outer wall surfaces (111,112), and being made from a deformable material such that as a result of movement of said inner wall surface (112) towards said accommodation space by the first biasing force, said pressure counteracting member (16) acquires an urging force which resists the first biasing force so as to hold said flashback chamber (11) in the ready position.

3. The intravenous catheter introducing device of Claim 1, **characterized in that** said flashback chamber (11) has an outer wall surface (111) and an inner wall surface (112) which is opposite to said outer wall surface (111), and which confronts said accommodation space, said rear end (121) defining an end opening communicated with said accommodation space, said intravenous catheter introducing device further comprising:
a plunger head (33) configured to be in a fluidly tight contact with, and to be slidable relative to, said inner wall surface (112) in the longitudinal direction;
a plunger stem (31) having a securing end (312) fixed to said plunger head (33), and an actuated end (311) which is opposite to said securing end (312) in the longitudinal direction, and which extends outwardly of said end opening so as to be actuated to move said plunger head (33) to slide along said inner wall surface (112) from a push-out position where said plunger head (33) is closer to said front wall portion (124), to a pull-in position where said plunger head (33) is remote from said front wall portion (124), thereby generating the first biasing force that biases said plunger head (33) towards said front wall portion (124) once said tip end (212) has pierced into the skin in search of the vein.

4. The intravenous catheter introducing device of Claim 3, **characterized in that** said pressure counteracting member includes
a retaining portion (14) which is fixed to said outer wall surface (111) adjacent to said rear end (121) of said flashback member (1),
an actuator (32) having a rear fixed end (325) which is fixed to said actuated end (311), a tongue portion (326) which extends from said rear fixed end (325) forwardly and which terminates at a front pushed end (321), and a plurality of retained regions (324) which are disposed on said tongue portion (326) and which are spaced apart from one another in the longitudinal direction, said tongue portion (326) being displaceable relative to said plunger stem (31) between a retained position, where a selected one of said retained regions (324) is engaged with said retaining portion (14) so as to resist against the first biasing force to thereby prevent said plunger head (33) from being shifted relative to said inner wall surface (112), and a released position, where said tongue portion (326) is released from said retaining portion (14) so as to permit sliding movement of said plunger head (33) along said inner wall surface (112).

5. The intravenous catheter introducing device of Claim 4, **characterized in that** said retaining portion (14) has a confining hole (141) extending therethrough in the longitudinal direction,
said rear fixed end (325) of said actuator (32) being integrally formed with said actuated end (311) of said plunger stem (31), said tongue portion (326) being disposed to extend through said confining hole (141), and being configured to acquire a second biasing force that biases the selected one of said retained regions (324) to the retained position, thereby placing said tongue portion (326) in the retained position.

6. The intravenous catheter introducing device of Claim 4, **characterized in that** said retaining portion (14) has an engaging slot (15) which is communicated with said confining hole (141) in a transverse direction relative to the longitudinal direction, said actuator (32) having a longitudinally extending reinforcing ridge (323) which is disposed on said tongue portion (326) to increase stiffness of said tongue portion (326) and which is configured to engage said engaging slot (15) when said tongue portion (326) is placed in the retained position.

7. The intravenous catheter introducing device of Claim 4, **characterized in that** said actuator (32) has a pair of limiting barriers (322) which are disposed between said retained regions (324) and said front pushed end (321), and which are configured to extend transversely to be blocked by said retaining portion (14) when said tongue portion (326) is in the released position so as to prevent excess rearward movement of said plunger head (33) relative to said inner wall surface (112).

8. The intravenous catheter introducing device of Claim 3, further **characterized by** a pair of lugs (13) which are disposed on and which extend from said outer wall surface (111) for gripping by a user's fingers so as to facilitate pulling of said plunger head (33) to the pull-in position.

9. The intravenous catheter introducing device of Claim 3, **characterized in that** said pressure counteracting member includes
a traction ring (41) which is sleeved on and which is in frictional engagement with said outer wall surface (111) of said flashback chamber (11) with a frictional force, and
a connecting bar (42) which interconnects said traction ring (41) and said actuated end (311) of said plunger stem (31) to permit said traction ring (41) to be moved with said plunger head (33) such that once said actuated end (311) is relieved of a manual force immediately after said plunger head (33) is pulled by the manual force against the frictional force to move from the push-out position to the pull-in position to create the reduced pressure, the frictional force can hold said traction ring (41) to said outer wall surface (111) against the pressure difference, thereby holding said plunger head (33) at the pull-in position.

10. The intravenous catheter introducing device of Claim 1, further **characterized by**:
an internal needle (28) disposed rearwardly of said rear connecting segment (211) in the longitudinal direction;
a needle seat (25) which is disposed to secure said rear connecting segment (211); and
a shell member (5) which is disposed rearwardly of said needle seat (25), and which extends rearwardly to surround said internal needle (28) and to terminate at a rear access end (521), thereby defining a receiving chamber (51) to detachably receive said flashback member (1) from said rear access end (521) such that when said flashback member (1) is received in said receiving chamber (51), said internal needle (28) is brought to pass through said front end (122) of said flashback member (1) so as to permit said needle cannula (21) to fluidly communicate said flashback chamber (11).

11. The intravenous catheter introducing device of Claim 10, further **characterized by** an air-permeable and liquid-impermeable partition member (53) which is disposed in said receiving chamber (51), and which is pierceable by said internal needle (28), said partition member (53) being configured to air-tightly engage and to be movable relative to said shell member (5) such that once said partition member (53) is pushed by said flashback member (1) to permit said internal needle (28) to pierce through said partition member (53), a subsequent pushing action of said flashback member (1) results in passing of said internal needle (28) through said front end (122) of said flashback member (1) so as to fluidly communicate said flashback chamber (11) with said needle cannula (21).

12. The intravenous catheter introducing device of Claim 11, further **characterized by** an annular barrier (525) which extends from said shell member (5) inwardly and radially such that said partition member (53) is prevented from being removed from said shell member (5) through said rear access end (521) while permitting said internal needle (28) to pass through said front end (122) of said flashback member (1).

13. The intravenous catheter introducing device of Claim 10, **characterized in that** said internal needle (28) is integrally formed with and extends rearwardly from said rear connecting segment (211) of said needle cannula (21), said needle seat (25) being integrally formed with said shell member (5).

14. The intravenous catheter introducing device of Claim 10, **characterized in that** said internal needle (28) has a first segment which extends into said receiving chamber (51) to be surrounded by said shell member (5), and a second segment opposite to said first segment,
said device further comprising a needle hub member (524) which extends forwardly from said shell member (5) and which surrounds and secures said second segment of said internal needle (28),
said needle seat (25) having a front securing portion (252) which is configured to secure said rear connecting segment (211), and a rear coupling portion (251) which is detachably sleeved on said needle hub member (524) such that said needle cannula (21) is fluidly communicated with said internal needle (28), said catheter hub (22) being detachably sleeved on said front securing portion.

15. The intravenous catheter introducing device of Claim 10, further **characterized by**:
a front sealing member (63) configured to be in air-tight engagement with said front wall portion (124) so as to seal said front end (122); and
a rear sealing member (64) configured to be in air-tight engagement with said rear wall portion (123) so as to seal said rear end (121), thereby confining said flashback chamber (11).

16. The intravenous catheter introducing device of Claim 15, **characterized in that** said rear sealing member (64) is disposed to be movable relative to said surrounding wall (12) in the longitudinal direction so as to vary the volume of said accommodation space in said flashback chamber (11) to create the reduced pressure therein.

17. The intravenous catheter introducing device of Claim 16, further **characterized by** a plunger (65) having a head portion (651) which is connected to said rear sealing member (64), and a shank portion (652) which extends rearwardly from said head portion (651) and outwardly of said rear end (121) such that said plunger (65) is pulled rearwardly to move said rear sealing member (64) towards said rear end (121) to generate the reduced pressure in said flashback chamber (11), said shank portion (652) having a weakening region (653) which is formed proximate to said head portion (651) for facilitating breaking of said shank portion (652).

18. The intravenous catheter introducing device of Claim 17, **characterized in that** said pressure counteracting member includes first and second engaging portions (125,654) which are disposed on said rear wall portion (123) adjacent to said rear end (121) and said head portion (651), respectively, and which are configured such that a force movement of said second engaging portion (654) over said first engaging portion (125) by virtue of rearwardly pulling said head portion (651) results in a press-fit engagement between said first and second engaging portions (125,654), thereby preventing a forward movement of said rear sealing member (64) and holding said flashback chamber (11) in the ready position.

19. The intravenous catheter introducing device of Claim 1, further **characterized by** a tubular needle seat (25) which is disposed to be surrounded by, and to be integrally formed with, said front wall portion (124), and which is configured to secure said rear connecting segment (211) of said needle cannula (21) to said front wall portion (124) while permitting said needle cannula (21) to be fluidly communicated with said flashback chamber (11).

20. The intravenous catheter introducing device of Claim 19, further **characterized by** a tip protector (24) which is detachably sleeved on said rear wall portion (123) for shielding said needle cannula (21), said catheter hub (22) and said tubular catheter (23).

21. The intravenous catheter introducing device of Claim 1, further **characterized by** a tubular needle seat (25) having a rear coupling portion (251) which is detachably sleeved on said front wall portion (124), and a front securing portion (252) which is configured to secure said rear connecting segment (211) of said needle cannula (21) such that said needle cannula (21) is fluidly communicated with said flashback chamber (11), said catheter hub (22) being detachably sleeved on said front securing portion (252).

22. The intravenous catheter introducing device of Claim 21, further **characterized by** a tip protector (24) which is detachably sleeved on said catheter hub (22) for shielding said needle cannula (21) and said tubular catheter (23), and which is configured to be detachably sleeved on said rear coupling portion of said tubular needle seat (25) when said catheter hub (22) is detached from said front securing portion.
